# FASCICULE DE BREVET EUROPEEN

(11) **EP 3 766 580 B1**
(45) Date de publication et mention de la délivrance du brevet: **08.12.2021**
(21) Numéro de dépôt: 20184841.3
(22) Date de dépôt: 09.07.2020
(51) Int. Cl.: B01L 3/00

(54) **DISPOSITIF MICRO-FLUIDIQUE DE PRÉPARATION ET D'ANALYSE D'UN ÉCHANTILLON BIOLOGIQUE**
MIKROFLUIDVORRICHTUNG ZUR VORBEREITUNG UND ANALYSE EINER BIOLOGISCHEN PROBE
MICROFLUIDIC DEVICE FOR PREPARING AND ANALYSING A BIOLOGICAL SAMPLE

(30) Priorité: 17.07.2019 FR 1908032
(43) Date de publication de la demande: 20.01.2021
(73) Titulaire: Commissariat à l'Energie Atomique et aux Energies Alternatives, 75015 Paris (FR)
(72) Inventeur: BORDY, Thomas, 38054 GRENOBLE Cedex 09 (FR); BOURDAT, Anne-Gaëlle, 38054 GRENOBLE Cedex 09 (FR); TOUTAIN, Rémi, 38054 GRENOBLE Cedex 09 (FR); PAULUS, Caroline, 38054 GRENOBLE Cedex 09 (FR)
(74) Mandataire: INNOV-GROUP

(56) Documents cités:
- EP-B1- 3 222 989
- US-A1- 2002 124 896
- US-A1- 2009 038 417
- US-A1- 2016 077 062
- DAFENG CHEN ET AL: "An integrated, self-contained microfluidic cassette for isolation, amplification, and detection of nucleic acids", BIOMEDICAL MICRODEVICES, KLUWER ACADEMIC PUBLISHERS, BO, vol. 12, no. 4, 17 avril 2010 (2010-04-17) , pages 705-719, XP019814145, ISSN: 1572-8781

## Description

### Domaine technique de l'invention

La présente invention se rapporte à un dispositif micro-fluidique de préparation et d'analyse d'un échantillon biologique contenant des espèces biologiques.

Ce dispositif permet notamment de réaliser toutes les étapes de préparation et d'analyse de l'échantillon dans un même support rigide.

### Etat de la technique

Depuis l'invention de la PCR ("Polymerase Chain Reaction") et même de la qPCR ("Quantitative PCR"), de nombreuses applications ont été développées autour de la biologie moléculaire pour détecter et identifier des organismes à partir de leurs acides nucléiques. Dans la plupart des cas, il s'agit de détection par PCR à l'aide d'amorces ADN spécifiques à une cible donnée (cellules rares dans le sang, virus dans les voies respiratoires ou encore bactéries dans des matrices alimentaires). Une étape de préamplification par culture des cellules recherchées est souvent nécessaire car le test n'est pas toujours assez sensible pour détecter de très faibles concentrations ou la matrice et le réactif de lyse cellulaire (ou bactérien, ou viral) dans laquelle se trouve l'échantillon est inhibitrice du test (ex : sang, fromage...). Il est alors nécessaire de diluer l'échantillon pour diminuer les inhibiteurs, ce qui entraîne une dilution des cellules ou de l'ADN cibles.

Actuellement, les tests connus sont réalisés en trois phases principales : une phase de mise en culture de l'échantillon, puis lyse des cellules pour rendre l'ADN accessible, puis une phase de purification ou dilution des acides nucléiques présents et d'amplification biomoléculaire. La phase de culture est peu coûteuse mais s'avère très longue (de 8h à 72h). La phase de libération de l'ADN et de purification/dilution pour l'amplification des acides nucléiques nécessite pour sa part de nombreuses manipulations, ce qui la rend difficile à appréhender par du personnel non qualifié. Elle peut certes être réalisée de manière automatisée mais les appareils connus sont souvent imposants et coûteux.

La détection de pathogènes dans un échantillon biologique est donc souvent réalisée en laboratoire en employant un matériel lourd et peu adapté à une analyse rapide sur le terrain. Pour rappel, la mise en place d'une telle détection sur un échantillon biologique peut nécessiter d'effectuer toutes les étapes suivantes :
- enrichissement dans un milieu de culture,
- concentration des espèces biologiques présentes dans l'échantillon,
- purification des espèces biologiques ciblées,
- lyse des espèces biologiques contenues dans l'échantillon pour casser lesdites espèces et libérer un matériel biologique à analyser,
- purification et ou dilution du matériel biologique,
- amplification biomoléculaire de type qPCR, LAMP, RPA, ou tout autre mode de détection par amplification biomoléculaire ou séquençage
- détection de l'amplification à chaque cycle par optique tel que par exemple fluorescence, colorimétrie, imagerie holographique, turbidimétrie, pHmétrie en liaison avec la réaction d'amplification.

Des dispositifs qui permettent de mettre en œuvre certaines des étapes décrites ci-dessus, notamment les étapes de concentration, de purification et de lyse mécanique, sont connus de l'état de la technique. La demande de brevet WO2015/181743A1 décrit notamment un tel dispositif. Dans celui-ci, la lyse mécanique est réalisée par cisaillement entre deux parois, l'une des deux parois ayant une surface d'appui rugueuse. Un tel dispositif permet essentiellement de réaliser le broyage et n'est pas adapté pour mettre en œuvre une analyse plus complète d'un échantillon biologique, l'analyse par détection optique devant être menée après transfert de l'échantillon en dehors du dispositif, ce qui peut entraîner une contamination de l'échantillon et fausser les résultats.

Il existe par ailleurs des solutions qui permettent de réaliser une détection de la présence de pathogènes par amplification et détection par colorimétrie ou turbidité. De telles solutions sont par exemple décrites dans les publications suivantes :
- « Visual detection of isothermal nucleic acid amplification using pH-sensitive dyes », Nathan A. Tanner et al - BioTechniques, Vol. 58, n°2, February 2015, pp. 59-68.
- « Colorimetric detection of loop-mediated isothermal amplification reaction by using hydroxy naphthol blue », Motoki Goto et al. - BioTechniques, Vol. 46, No. 3, March 2009, pp. 167-172.
- « Loop-Mediated Isothermal Amplification Assay for Rapid Detection of Common Strains of Escherichia coli », Hill J, Beriwal S, Chandra I, et al. - Journal of Clinical Microbiology. 2008;46(8):2800-2804. doi:10.1128/JCM.00152-08.
- « Visual Detection of Norovirus Genogroup II by Reverse Transcription Loop-Mediated Isothermal Amplification with Hydroxynaphthol Blue Dye », Jianming, Ziqian Xu, Kai Nie, Xiong Ding, Li Guan, Ji Wang, Yuying Xian, Xiyang Wu , Xuejun Ma - Food and Environmental Virology, September 2014, Volume 6, Issue 3, pp 196-201.

Le document US 2002/124896 A1 décrit des systèmes microfluidiques modulaires comportant éventuellement un module de filtration. Le document US 2016/077062 A1 concerne un dispositif d'injection microfluidique mettant en œuvre l'insertion d'un élément. Le brevet EP3222989B1 décrit pour sa part un dispositif micro-fluidique qui permet également de réaliser certaines des étapes décrites ci-dessus, notamment la concentration, la lyse et la détection par lecture optique.

Ce dernier dispositif est donc particulièrement complet mais il ne permet pas de mettre en œuvre toutes les étapes décrites ci-dessus. Par ailleurs, même si certaines étapes peuvent rester optionnelles, il est utile de disposer d'un dispositif plus polyvalent qui permet de réaliser toutes les étapes, sans transfert de l'échantillon en dehors du dispositif et donc sans risque de contamination.

### Exposé de l'invention

Ce dispositif micro-fluidique polyvalent permet ainsi la préparation et l'analyse d'un échantillon biologique et comporte :
- Un unique support rigide,
- Une première unité comprenant une première chambre de volume non nul délimitée par des parois du support, un filtre séparant ladite première chambre en un premier espace et un deuxième espace, un premier canal réalisé dans ledit support débouchant à une extrémité sur une surface dudit support et à une autre extrémité dans ledit premier espace et un deuxième canal réalisé dans ledit support et débouchant à une extrémité sur une surface dudit support et à une autre extrémité dans ledit deuxième espace,
- Une deuxième unité comprenant une deuxième chambre réalisée dans ledit support rigide et délimitée au moins partiellement par une paroi transparente dudit support, un troisième canal réalisé dans ledit support et débouchant à une extrémité sur une surface dudit support et à une autre extrémité dans ladite deuxième chambre,
- Un premier canal de transfert fluidique entre la première chambre et la deuxième chambre, réalisé dans ledit support et débouchant d'une part dans ladite deuxième chambre et d'autre part en un premier nœud de dérivation dans ledit deuxième canal,
- Des premiers moyens d'aiguillage de flux agencés pour sélectionner la liaison de la première chambre :
   ∘ vers l'extérieur uniquement via le deuxième canal uniquement ou,
   ∘ vers la deuxième chambre uniquement à travers le premier canal de transfert.

Selon une particularité, la première unité comporte une surface d'appui rugueuse réalisée au fond de sa première chambre.

Selon une autre particularité, la première chambre est fermée par une membrane déformable.

Selon une autre particularité, la première unité est configurée pour mettre en œuvre une ou plusieurs des étapes suivantes d'un procédé de préparation et d'analyse d'un échantillon biologique :
- Concentration des espèces biologiques présentes dans un échantillon biologique,
- Lavage pour purification des espèces biologiques,
- Réception d'un milieu de culture,
- Culture des espèces biologiques,
- Lyse des espèces biologiques en vue de libérer un matériel biologique,
- Séparation du matériel biologique.

Selon une autre particularité, la deuxième unité est configurée pour mettre en œuvre une ou plusieurs des étapes suivantes d'un procédé de préparation et d'analyse d'un échantillon biologique :
- Culture des espèces biologiques,
- Suivi optique de croissance lors de ladite étape de culture,
- Détection de présence de pathogènes dans le matériel biologique séparé, par amplification biomoléculaire.

Selon une autre particularité, le dispositif comporte une première membrane hydrophobe obturant le troisième canal.

Selon une autre particularité, le dispositif comporte :
- Une troisième unité comprenant une troisième chambre réalisée dans ledit support rigide et délimitée au moins partiellement par une paroi transparente dudit support, un quatrième canal réalisé dans ledit support et débouchant à une extrémité sur une surface dudit support et à une autre extrémité dans ladite troisième chambre.

Selon une autre particularité, le dispositif comporte :
- Un deuxième canal de transfert fluidique entre la première chambre (10) et la troisième chambre, réalisé dans ledit support et débouchant d'une part dans ladite troisième chambre et d'autre part en un deuxième nœud de dérivation dans ledit premier canal.

Selon une autre particularité, le dispositif comporte des deuxièmes moyens d'aiguillage de flux agencés pour sélectionner la liaison de la première chambre :
- vers l'extérieur uniquement via le premier canal uniquement ou,
- vers la troisième chambre uniquement à travers le deuxième canal de transfert.

Selon une autre particularité, la troisième unité est configurée pour mettre en œuvre l'étape suivante d'un procédé de préparation et d'analyse d'un échantillon biologique :
- Détection de présence de pathogènes dans le matériel biologique séparé, par amplification biomoléculaire.

Selon une autre particularité, le dispositif comporte une deuxième membrane hydrophobe obturant le quatrième canal.

La réaction de type qPCR consiste en une amplification d'une séquence d'ADN ou d'ARN ciblée (représentative d'un organisme en particulier) couplée à un intercalant ou une sonde produisant une fluorescence détectable par un appareil optique en cas d'amplification de cette séquence. Ainsi, si le niveau de fluorescence augmente durant la réaction, cela signifie que la réaction d'amplification se produit et que donc l'ADN ou l'ARN de l'organisme cible était bien présent. Néanmoins, en cas d'absence de réaction, il faut être capable d'affirmer que cela est dû à l'absence de l'organisme recherché et non à une inhibition de la réaction d'amplification, ce qui donnerait lieu à un faux négatif. Les enzymes responsables de la réaction d'amplification sont en effet sensibles à de nombreux inhibiteurs apportés par l'échantillon analysé.

Afin de garantir que l'absence d'amplification signifie bien l'absence de la cible, des contrôles internes de réactions sont mis en place. La plupart du temps, il s'agit d'une autre cible d'ADN ajoutée volontairement au test qui va être amplifiée simultanément avec l'échantillon d'intérêt. Il faut alors être capable de le discriminer les deux réactions. Plusieurs stratégies sont utilisées dans l'industrie :
- L'une d'elles pourrait être d'utiliser une partie de l'échantillon pour réaliser le contrôle en parallèle, en tant que réaction indépendante. Ceci oblige à fractionner l'échantillon initial entrainant une perte de la sensibilité/représentativité du test. L'avantage est qu'il est possible d'utiliser n'importe quelle technique de détection d'amplification pour ce contrôle (exemple : intercalant ADN fluorescent).
- Afin de ne pas diviser l'échantillon, une autre stratégie consiste à réaliser le contrôle dans la même réaction que la séquence ciblée : avec par exemple l'utilisation de sondes ADN spécifiques de la séquence cible et en parallèle des sondes ADN spécifiques de la séquence contrôle (avec un fluorochrome différent pour chaque sonde). Cette solution permet de tester l'intégralité de l'échantillon mais n'est pas compatible avec toutes les techniques de détection, notamment avec l'utilisation d'agent intercalent non spécifique, d'une séquence ou de tout autre mode de détection non spécifique de séquence (colorimetrie, pH metrie..).

La demande de brevet EP0586112A2 et le brevet US6312930B1 décrivent chacun une méthode de détection permettant d'éliminer les faux négatifs, par ajout d'une cible de contrôle.

L'invention vise donc également à conférer à la deuxième chambre une architecture particulière, en façonnant son volume interne.

Selon une autre particularité, la deuxième chambre comporte ainsi au moins une alcôve destinée à recevoir un composé de contrôle interne de réaction.

Selon une autre particularité, la deuxième chambre est réalisée en plusieurs strates superposées et en ce que ladite alcôve est réalisée dans l'une desdites strates uniquement.

L'invention concerne également un procédé de préparation et d'analyse d'un échantillon biologique contenant des espèces biologiques, ledit procédé étant mis en œuvre à l'aide d'un dispositif micro-fluidique tel que défini ci-dessus, dans lequel :
- La première unité est configurée pour mettre en œuvre une ou plusieurs des étapes suivantes dudit procédé :
   ∘ Concentration des espèces biologiques présentes dans un échantillon biologique,
   ∘ Lavage pour purification des espèces biologiques,
   ∘ Réception d'un milieu de culture,
   ∘ Culture des espèces biologiques,
   ∘ Lyse des espèces biologiques en vue de libérer un matériel biologique,
   ∘ Séparation du matériel biologique,
- La deuxième unité est configurée pour mettre en œuvre une ou plusieurs des étapes suivantes dudit procédé :
   ∘ Culture des espèces biologiques,
   ∘ Suivi optique de croissance lors de ladite étape de culture,
   ∘ Détection de présence de pathogènes dans le matériel biologique séparé, par amplification biomoléculaire,
- La troisième unité est configurée pour mettre en œuvre l'étape suivante dudit procédé :
   ∘ Détection de présence de pathogènes dans le matériel biologique séparé, par amplification biomoléculaire.

### Brève description des figures

D'autres caractéristiques et avantages vont apparaître dans la description détaillée qui suit faite en regard des dessins annexés dans lesquels :
- La figure 1 illustre les différentes configurations possibles d'un procédé de préparation et d'analyse d'un échantillon contenant des espèces biologiques.
- Les figures 2A à 2C représentent trois variantes de réalisation du dispositif de l'invention.
- Les figures 3A à 3D représentent les différentes étapes de préparation et d'analyse mises en œuvre avec le dispositif de la figure 2A.
- Les figures 4A à 4H représentent les différentes étapes de préparation et d'analyse mises en œuvre avec le dispositif de la figure 2B.
- Les figures 5A à 5F représentent les différentes étapes de préparation et d'analyse mises en œuvre avec le dispositif de la figure 2C.
- La figure 6 représente, vu en éclaté, un exemple de réalisation du dispositif de l'invention, dans la variante de la figure 2B.
- La figure 7 représente un exemple d'une chambre d'amplification pouvant être employée dans le dispositif de l'invention.
- Les figures 8A à 8C représentent plusieurs variantes de réalisation de la strate inférieure de la chambre d'amplification, pouvant être employée dans le dispositif de l'invention.

### Description détaillée d'au moins un mode de réalisation

Le dispositif micro-fluidique de l'invention est destiné à l'analyse d'un échantillon biologique. Cet échantillon biologique se présente par exemple sous la forme d'un fluide qui contient des espèces biologiques contenant un matériel biologique à étudier.

Par espèces biologiques, on entend notamment des micro-organismes, des cellules, des spores, virus... Par matériel biologique à étudier, on entend par exemple des molécules d'acide nucléique (ARN, ADN) issues d'une cellule, des protéines, des lipopolysaccharides (LPS), des acides lipotéichoïques (LTA)...

Par fluide, on entend notamment un liquide, un gaz... Le liquide pourra présenter différents degrés de viscosité et pourra par exemple se présenter sous la forme d'une pâte ou d'un gel.

Dans la suite de la description, les termes "inférieur", "supérieur", "haut" et "bas" employés sont à comprendre en prenant comme référence un axe principal (X) qui est vertical.

Dans la suite de la description, les termes "externe", "extérieur", "interne", "intérieur", doivent être compris en prenant comme référence les chambres du dispositif qui seront décrites ci-dessous.

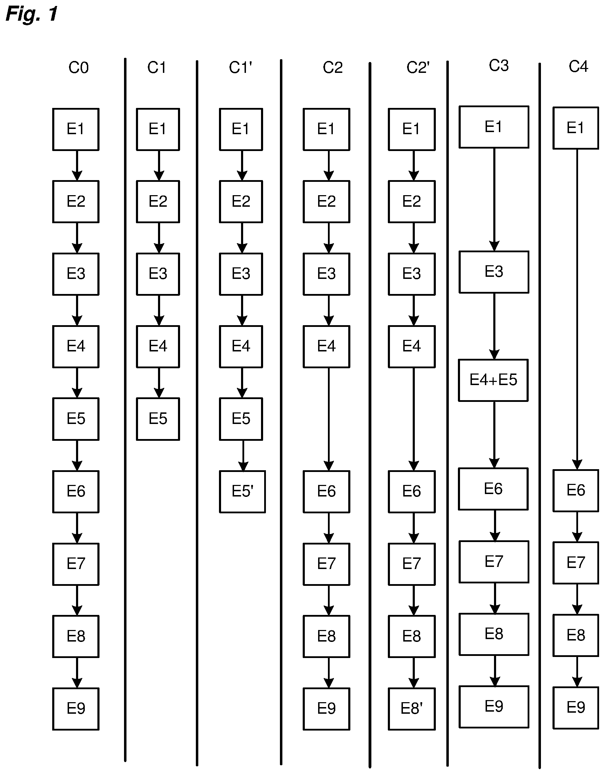

En référence à la figure 1, l'analyse complète (colonne C0) d'un échantillon biologique peut comporter les étapes suivantes réalisées de manière successive :
- E1 : Concentration des espèces biologiques présentes dans l'échantillon biologique,
- E2 : Lavage pour purification, pour élimination des interférents de culture,
- E3 : Apport d'un milieu de culture,
- E4 : Culture des espèces biologiques,
- E5 : Suivi optique de croissance lors de la culture et comptage des colonies,
- E6 : Lavage, pour élimination des inhibiteurs de PCR,
- E7 : Lyse mécanique des espèces biologiques présentes dans l'échantillon en vue d'en extraire un matériel biologique à étudier,
- E8 : Séparation entre le matériel biologique à étudier et les polluants présents,
- E9 : Une détection de présence de pathogènes dans le matériel biologique par amplification biomoléculaire de type qPCR, LAMP, RPA et détection optique tel que par exemple fluorescence, colorimétrie, imagerie holographique, turbidimétrie, pHmétrie en liaison avec la réaction d'amplification.

Lors de l'étape de concentration, l'échantillon biologique, par exemple sous forme liquide, comprenant les espèces biologiques, est injecté dans une chambre pour passer à travers un filtre. La partie liquide de l'échantillon et toutes les particules/molécules qui passent à travers le filtre sont récupérées par un canal d'évacuation et écartées de l'analyse. Les espèces biologiques sont alors concentrées dans un espace de la chambre.

Une solution de lavage/rinçage peut être injectée pour laver les espèces biologiques présentes dans la chambre.

Un milieu de culture est injecté pour permettre la culture des espèces biologiques.

L'étape de suivi de croissance permet, par lecture optique, de suivre la croissance cellulaire lors de l'étape de culture.

La lyse mécanique des espèces biologiques est mise en œuvre pour broyer les espèces biologiques présentes dans l'échantillon contre une surface d'appui rugueuse. Une fois la lyse mécanique effectuée, on dispose d'un matériel biologique à étudier, formé par exemple de molécules d'ADN et de polluants.

La séparation entre le matériel biologique à étudier et les polluants est réalisée en injectant une solution liquide contenant des réactifs d'amplification, pour éluer le matériel biologique à étudier. Une partie de la solution liquide injectée emporte ainsi le matériel biologique à étudier, par exemple les molécules d'ADN, qui passe à travers le filtre.

Une fois que la séparation entre les polluants et le matériel biologique à étudier est effectuée, la réaction d'amplification du matériel biologique permet de détecter la présence d'un pathogène dans le matériel biologique qui a été séparé. La réaction d'amplification est mise en œuvre en ajoutant un mélange d'amplification et en chauffant une chambre dans laquelle est placée l'échantillon. La température à laquelle est chauffée la chambre dépend du type de réaction d'amplification mise en œuvre. Il pourra s'agir de tous types de réaction d'amplification, par exemple LAMP ("Loop-Mediated *Isothermal* Amplification"), PCR ("Polymerase Chain Reaction"), NASBA ("Nucleic Acid Sequence Based Amplification"), RPA ("Recombinase Polymerase Amplification")... Pour une amplification de type LAMP, le chauffage est réalisé à une température avantageusement comprise entre 60°C et 65°C. Cette réaction permet d'amplifier les molécules du matériel biologique à détecter, par exemple les molécules d'ADN. Lors de la réaction d'amplification du matériel biologique, il s'agit de détecter si un pathogène est présent. Pour cela, il est possible d'employer différentes méthodes, comme par exemple la colorimétrie, la fluorescence, l'électrochimie, la pHmétrie, la mesure de turbidité. Toute autre méthode de détection pourrait être envisagée. Pour une méthode de détection de type PHmétrie, des électrodes de détection de pH pourraient être intégrées au dispositif.

Certaines des étapes ci-dessus sont cependant optionnelles, le procédé d'analyse peut donc prendre différentes configurations possibles.

Dans une première configuration C1 (figure 1), le procédé peut comporter les étapes suivantes :
- E1 : Concentration,
- E2 : Lavage,
- E3 : Apport d'un milieu de culture,
- E4 : Culture
- E5 : Suivi optique de croissance lors de la culture et comptage des colonies,

Dans une configuration C1' reprenant les étapes E1 à E5 de la première configuration, une étape E5' peut être ajoutée après l'étape E5 afin de collecter des bactéries pour traitement.

Dans une deuxième configuration C2 (figure 1), le procédé peut comporter les étapes suivantes :
- E1 : Concentration,
- E2 : Lavage,
- E3 : Apport d'un milieu de culture,
- E4 : Culture,
- E6 : Lavage,
- E7 : Lyse,
- E8 : Séparation,
- E9 : Détection par amplification.

De même, dans une configuration C2' reprenant les étapes E1 à E8 de la configuration C2, une étape E8', venant remplacer l'étape E9, peut être ajoutée. Cette étape consiste à ressortir l'ADN séparé pour détection/stockage, notamment en vue d'un séquençage d'ADN.

Dans une troisième configuration C3 (figure 1), le procédé peut comporter les étapes suivantes :
- E1 : Concentration,
- E3 : Apport d'un milieu de culture,
- E4+E5 : Culture/Suivi de croissance,
- E6 : Lavage,
- E7 : Lyse,
- E8 : Séparation,
- E9 : Détection par amplification.

Dans une dernière configuration C4, il est également possible de se passer des étapes E2 à E5. On a ainsi :
- E1 : Concentration,
- E6 : Lavage, élimination des inhibiteurs de PCR,
- E7 : Lyse,
- E8 : Séparation,
- E9 : Détection par amplification.

Dans cette configuration C4, la variante incluant l'étape E8' est également envisageable, en vue de ressortir l'ADN libéré.

L'invention vise à proposer un dispositif micro-fluidique dont l'architecture est adaptée pour mettre en œuvre au moins deux des configurations décrites ci-dessus.

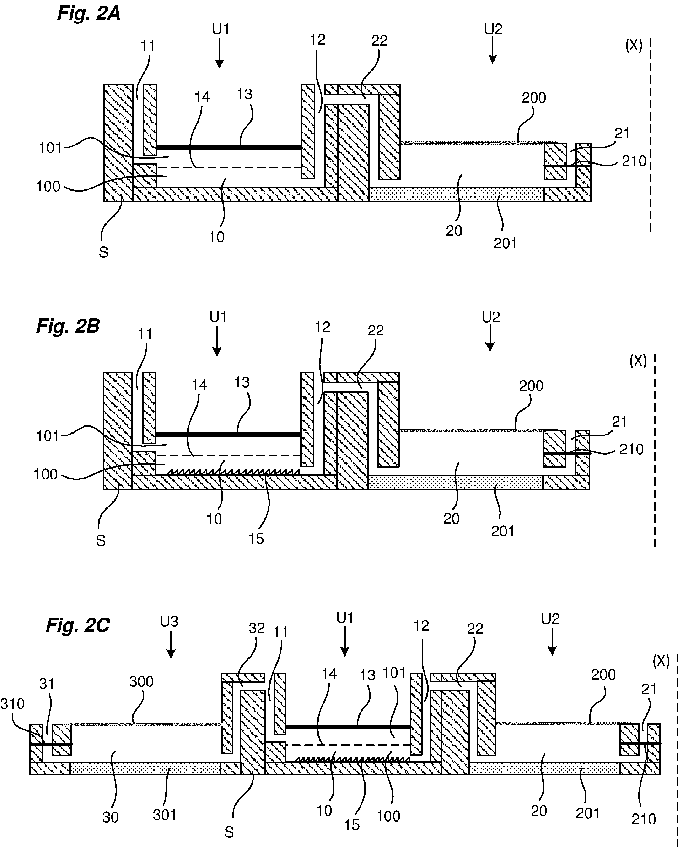

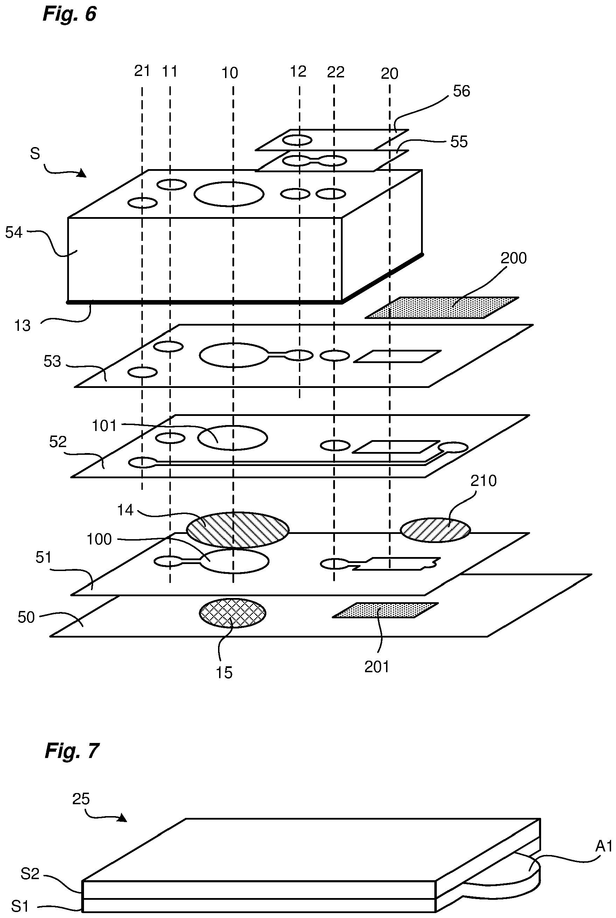

En référence aux figures 2A à 2C, le dispositif micro-fluidique comporte un unique support S rigide. La figure 6 décrite ci-dessous donne un exemple de réalisation du dispositif.

Ce support S rigide intègre un réseau micro-fluidique adapté à la mise en œuvre des étapes du procédé d'analyse. On verra que le réseau micro-fluidique peut prendre différentes architectures selon la configuration du procédé d'analyse qui est mise en œuvre.

Le support S comporte avantageusement une paroi inférieure plane et une architecture à plusieurs couches superposées suivant ledit axe principal, empilées sur sa paroi inférieure.

Le réseau micro-fluidique du dispositif comporte deux unités U1, U2 ou trois unités U1, U2, U3 employées chacune pour la mise en œuvre d'une ou plusieurs des étapes du procédé d'analyse, selon la configuration du procédé qui est choisie.

Dans le dispositif de l'invention la culture des espèces biologiques est réalisée en couche mince, c'est-à-dire avec un volume pouvant aller de 1 µL à 1 mL. L'avantage de réaliser une culture en couche mince est que les colonies peuvent être visibles beaucoup plus rapidement (durée de l'ordre de 2 à 3 heures) qu'avec une culture réalisée dans un mode classique. En combinant une étape de concentration des cellules et une culture en couche mince, il est ainsi possible d'analyser des échantillons très faiblement chargés en cellules. On pourra ainsi par exemple analyser des volumes importants d'eau, en vue de contrôler le niveau de contamination.

Par ailleurs, grâce au dispositif, il est possible de réaliser toute l'étape de purification des molécules d'ADN puis leur transfert vers une autre chambre pour amplification dans sa totalité, sans étape de dilution et sans aucun risque de contamination.

Dans les trois variantes de réalisation proposées et représentées sur les figures 2A à 2C, La première unité U1 comporte une première chambre 10 ménagée dans ledit support. Cette chambre 10 présente un volume non nul et est délimitée par des parois du support S.

La première unité U1 comporte un premier canal 11 ménagé dans le support pour injecter des fluides dans la première chambre 10 ou pour évacuer des fluides en dehors de cette première chambre. Le premier canal 11 comporte une première extrémité comportant une ouverture ménagée par exemple à travers une paroi supérieure du support S et une deuxième extrémité qui débouche dans ladite première chambre 10. La première extrémité du premier canal est par exemple agencée verticalement et sa deuxième extrémité débouche par exemple horizontalement dans la première chambre 10.

La première unité U1 comporte un deuxième canal 12. Ce deuxième canal 12 comporte également une première extrémité qui communique avec l'extérieur, formant une ouverture réalisée par exemple à travers une paroi supérieure du support S et une deuxième extrémité qui communique avec l'espace formé par la première chambre 10. Par ce deuxième canal 12, on peut également injecter des fluides dans ladite première chambre ou évacuer des fluides en dehors de ladite première chambre. Sa première extrémité est par exemple agencée verticalement et sa deuxième extrémité horizontalement. La première chambre 10 est placée entre le premier canal 11 et le deuxième canal 12.

Vers le haut, la première chambre 10 peut être fermée par une membrane 13 souple et étirable. Au niveau de la première chambre, une paroi supérieure du support comporte ainsi une ouverture qui est recouverte de manière hermétique par ladite membrane 13. La membrane 13 est ainsi ancrée dans le support par toute solution de fixation adaptée, par exemple par collage. Cette membrane 13 sera par exemple composée d'un film, par exemple de type MicroAmp, 3M (marques déposées), d'épaisseur, de dimensions et de constitution adaptées pour se déformer de manière hyper-élastique, par rapport à ses points d'ancrage, au moins jusqu'au fond de la première chambre.

La membrane 13 est apte à se déformer de manière réversible entre plusieurs configurations. Elle peut s'étirer par déformation hyper-élastique vers l'extérieur du support S, se rétracter à l'intérieur de la première chambre 10 par compression, ou être au repos. Par matériau hyper-élastique, on entend un matériau capable de présenter une surface apte à passer d'une première superficie à une deuxième superficie, la deuxième superficie étant égale à au moins 5 fois la première superficie, par exemple à 10 fois ou même à 50 fois la première superficie.

La première unité U1 comporte également un filtre 14 transversal agencé dans ladite première chambre 10 et séparant ladite première chambre 10 en deux espaces 100, 101. Les deux espaces sont par exemple superposés et désignés ainsi espace inférieur 100 situé sous le filtre 14 et espace supérieur 101 situé au-dessus du filtre 14. Ce filtre 14 est préférentiellement réalisé en tout ou partie sous la forme d'un film souple et fin, tiré dans l'espace formé par la chambre de manière à ne permettre le passage d'un espace à l'autre que par les pores du filtre 14. Le film présente une déformabilité élastique lui permettant de s'étirer lors de l'exercice d'une force d'appui dans une direction sensiblement verticale, cette déformabilité élastique ayant un niveau suffisant pour atteindre le fond de la chambre 10. Le filtre 14 présente un diamètre moyen de pores compris entre 0.2 µm et 50 µm, par exemple compris entre 0.2 µm et 1 µm pour la séparation de microorganismes. Le diamètre des pores est bien entendu adapté pour assurer une séparation entre différentes espèces biologiques présentes dans l'échantillon. Le filtre 14 sera par exemple composé d'un film d'épaisseur, de dimensions et de constitution adaptée pour se déformer jusqu'au fond de la chambre 10 par rapport à ses points d'ancrage. Il pourra comporter les mêmes caractéristiques d'hyper-élasticité que la membrane.

Selon une particularité, le premier canal 11 débouche dans l'espace supérieur 101 de la première chambre 10 et le deuxième canal 12 débouche dans l'espace inférieur 100 de la première chambre 10. Les embouchures des deux canaux sont donc séparées par le filtre 14 agencé dans la chambre.

En référence aux figures 2B et 2C, la première unité U1 peut avantageusement comporter une surface d'appui rugueuse 15 agencée sur le fond de la première chambre 10. Cette surface d'appui rugueuse 15 s'étend sur une partie majoritaire du fond de la première chambre. Elle comporte un paramètre de rugosité de surface moyen compris entre 0.1 µm et 10 µm, préférentiellement compris entre 0.2 µm et 3 µm. Cette surface d'appui rugueuse 15 est destinée à permettre une lyse mécanique des espèces biologiques présentes dans un échantillon biologique placé dans le dispositif. Préférentiellement, la lyse mécanique est réalisée en broyant lesdites espèces biologiques, par abrasion sur ladite surface d'appui rugueuse. L'opération de broyage est mise en œuvre par un mouvement de friction des espèces biologiques contre la surface d'appui rugueuse, en employant un organe de broyage adapté. Cet organe sera par exemple une spatule ou une tige, par exemple en matériau plastique ou métallique. Cet organe est appliqué de l'extérieur de la chambre 10 et son extrémité est appliquée contre la surface externe de la membrane 13 de manière à étirer la membrane 13 et le filtre 14 vers le fond de la première chambre 10 et ainsi frictionner les espèces biologiques présentes dans un échantillon contre la surface d'appui rugueuse 15.

La deuxième unité U2 du dispositif comporte pour sa part une deuxième chambre 20 de volume non nul, délimitée par des parois du support S. La deuxième unité U2 comporte également un troisième canal 21 ménagé dans ledit support. Ce troisième canal 21 comporte une première extrémité comportant une ouverture ménagée par exemple à travers une paroi supérieure du support et une deuxième extrémité qui débouche uniquement dans ladite deuxième chambre 20. La première extrémité de ce troisième canal 21 est par exemple agencée verticalement et sa deuxième extrémité débouche par exemple horizontalement dans la deuxième chambre 20. La première extrémité de ce troisième canal est par exemple obturée par une membrane 210 hydrophobe, c'est-à-dire imperméable au liquide mais perméable au gaz tel que l'air. Cette membrane 210 hydrophobe peut être réalisée dans un matériau de type PTFE (Polytétrafluoroéthylène).

Deux parois transversales du support, avantageusement une paroi supérieure 200 et une paroi inférieure 201 parallèles, délimitant partiellement la deuxième chambre 20, sont réalisées dans un matériau transparent, permettant ainsi de réaliser une lecture optique à travers le volume de la deuxième chambre. Par le terme "transparent", on entend que le matériau employé est au moins partiellement transparent à la lumière visible, de manière à laisser passer au moins 80% de cette lumière. Il faut ainsi comprendre qu'il sera suffisamment transparent pour voir l'intérieur de la chambre. La paroi inférieure peut être réalisée en verre et la paroi supérieure peut être formée d'un adhésif amovible collé pour fermer ladite deuxième chambre du côté supérieur.

Selon une particularité de l'invention, le dispositif comporte également un premier canal de transfert 22 ménagé dans ledit support. Ce premier canal de transfert 22 est destiné à relier la première chambre 10, plus précisément son espace inférieur 100, à la deuxième chambre 20.

Le premier canal de transfert 22 comporte une première extrémité débouchant directement dans le deuxième canal 12, formant ainsi un nœud de dérivation sur ce deuxième canal 12. Il comporte une deuxième extrémité débouchant directement dans la deuxième chambre.

Le dispositif comporte en outre des moyens d'aiguillage pouvant être par exemple agencés sur le deuxième canal 12 pour sélectionner la liaison de la première chambre :
- vers l'extérieur uniquement via le deuxième canal uniquement ou,
- vers la deuxième chambre uniquement à travers le premier canal de transfert.

Ces moyens d'aiguillage peuvent consister en un cône 120 amovible creux se présentant sous la forme d'un entonnoir. Lorsqu'il est inséré par sa pointe dans le deuxième canal 12, il permet la communication entre l'extérieur et la première chambre et sa paroi obture l'entrée du premier canal de transfert 22, réalisée au niveau du nœud de dérivation. Lorsqu'il est retiré, la première extrémité du deuxième canal 12 peut être obturée, par exemple à l'aide d'un adhésif 121 appliqué sur une surface du support et la liaison entre le premier canal de transfert 22 et le deuxième canal 12 est alors ouverte, permettant à un fluide de circuler entre la première chambre 10 et la deuxième chambre 20.

Bien entendu, les moyens d'aiguillage peuvent être réalisés selon d'autres variantes de réalisation. Le principe général étant de pouvoir accéder à la première chambre en obturant le canal de transfert ou permettre une liaison entre la première chambre et la deuxième chambre. Il peut ainsi s'agir d'un simple clapet qui :
- Dans une première position, permet d'autoriser l'accès au-deuxième canal en obturant l'embouchure du canal de transfert 22 au niveau du nœud de dérivation,
- Dans une deuxième position, permet d'ouvrir la liaison entre le deuxième canal 12 et le canal de transfert 22.

Dans une architecture à uniquement deux unités U1, U2 telles que décrites ci-dessus, le procédé peut être mis en œuvre selon la première configuration ou la deuxième configuration décrite ci-dessus.

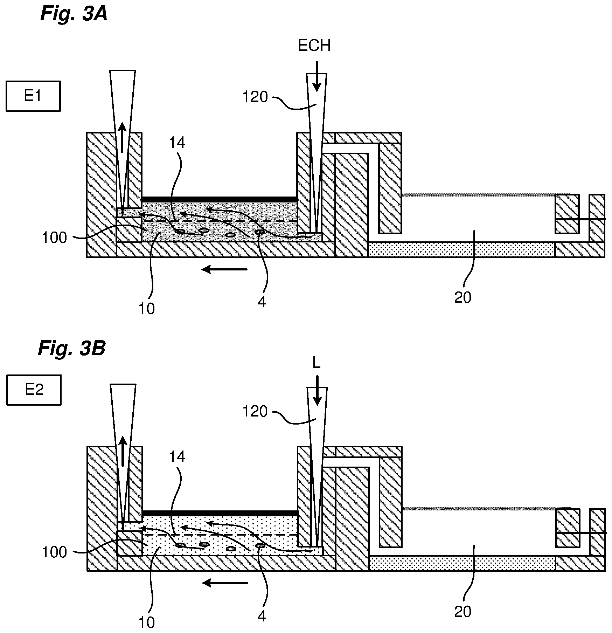

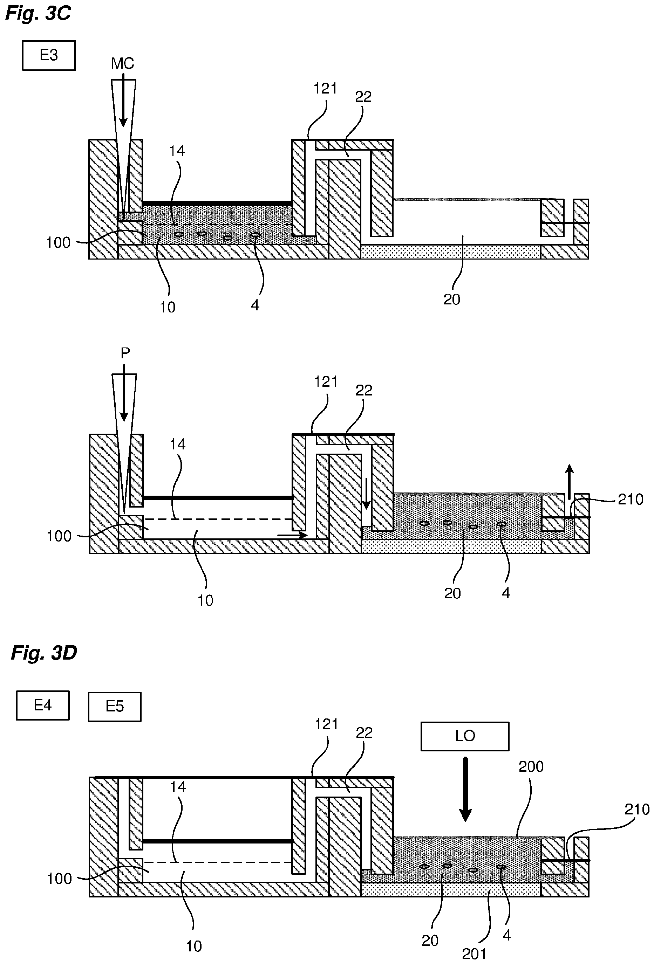

Dans la première configuration C1 du procédé mise en œuvre dans le dispositif de la figure 2A et en référence aux figures 3A à 3D, les étapes sont alors les suivantes :
- E1 : L'échantillon liquide ECH est injecté par le deuxième canal 12, par exemple à l'aide d'une pipette vers la première chambre 10 à travers le cône 120. Le cône 120 est agencé dans le deuxième canal à une profondeur suffisante pour obturer l'entrée du premier canal de transfert 22, au niveau du nœud de dérivation. Dans le même temps, le premier canal 11 est ouvert sur l'extérieur pour remplir le rôle d'évent lors du remplissage de la première chambre 10 et pour évacuer le liquide filtré. Lors de l'injection, sous l'effet de la pression d'injection, l'échantillon est filtré par le filtre 14. Les espèces biologiques 4 d'intérêt sont maintenues dans l'espace inférieur 100 de la première chambre 10 et le reste est évacué vers l'extérieur du dispositif par le premier canal 11.
- E2 : Dans la même configuration, l'étape de lavage (optionnelle) est ensuite mise en œuvre. Un liquide de lavage L est injecté par le deuxième canal 12, à travers le cône, pour purifier les espèces biologiques 4 capturées dans l'espace inférieur 100 de la première chambre 10. Ce liquide de lavage est évacué à travers le premier canal 11.
- E3 : Un milieu de culture MC est injecté par le premier canal 11 pour pénétrer dans la première chambre 10. L'entrée/sortie du deuxième canal 12 est fermée par l'adhésif 121 et le premier canal de transfert 22 est ouvert, permettant une communication étanche, sans risque de contamination, entre la première chambre 10 et la deuxième chambre 20.
- E3 : Les espèces biologiques 4 dans leur milieu de culture MC sont transférés vers la deuxième chambre 20 via le premier canal de transfert 22. Pour le transfert, une pression d'air P peut être appliquée dans le premier canal 11. Le troisième canal 21 est obturé grâce à la membrane 210 hydrophobe, permettant à l'air de s'évacuer lors du transfert et d'éviter au liquide de s'échapper. L'étape de culture peut durer 16 heures, en chauffant la deuxième chambre 20 à une température de 37°C.
- E4 - E5 : Un dispositif de lecture optique LO est activé pour réaliser un suivi de la culture des espèces biologiques présentes dans la deuxième chambre 20. La lecture optique est permise grâce à la transparence des deux parois 200, 201 parallèles délimitant la deuxième chambre 20.

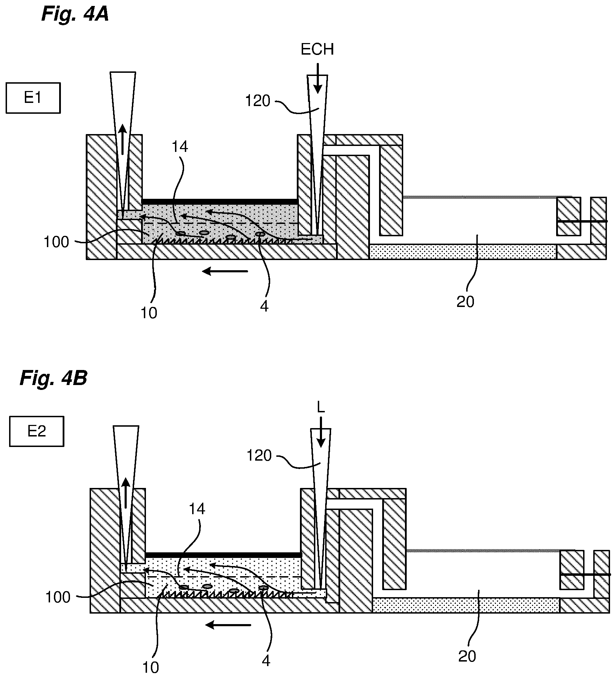

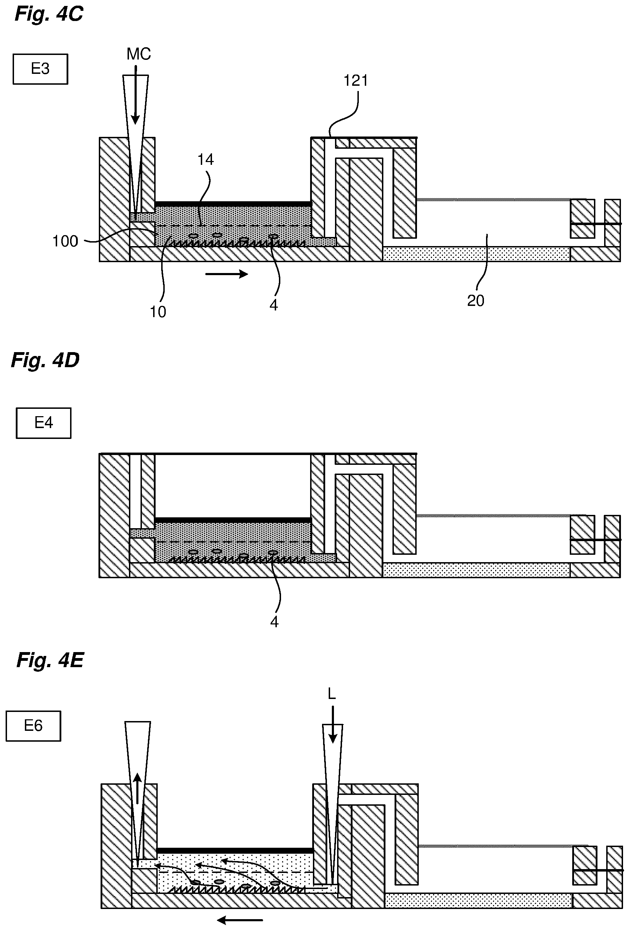

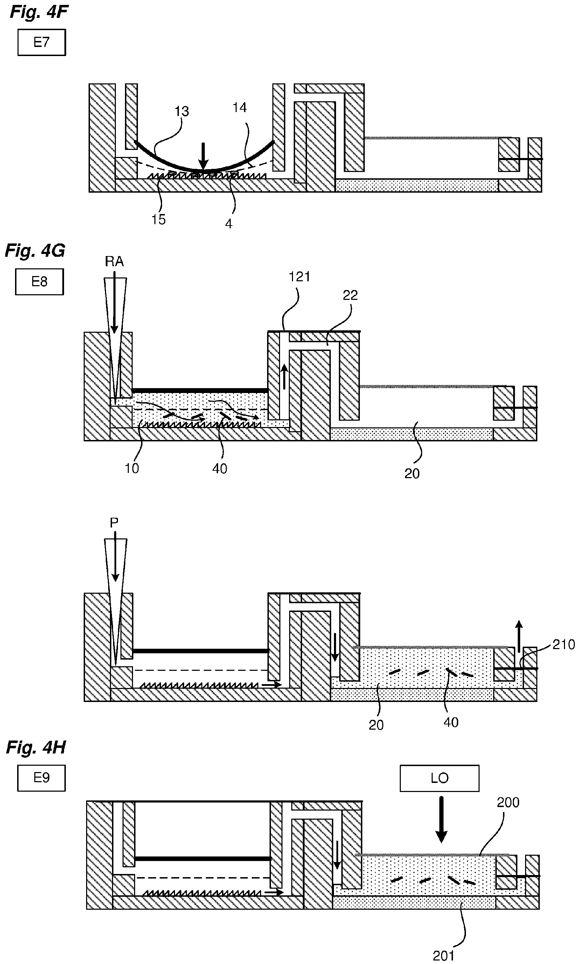

Dans la deuxième configuration C2 du procédé, mise en œuvre dans le dispositif de la figure 2B, et en référence aux figures 4A à 4H, les étapes sont alors les suivantes :
- E1 : L'échantillon liquide ECH est injecté par le deuxième canal 12, par exemple à l'aide d'une pipette vers la première chambre 10 à travers le cône 120. Le cône est agencé dans le deuxième canal 12 à une profondeur suffisante pour obturer l'entrée du premier canal de transfert 21, au niveau du nœud de dérivation. Dans le même temps, le premier canal 11 est ouvert sur l'extérieur pour remplir le rôle d'évent lors du remplissage de la première chambre 10 et pour évacuer le liquide filtré. Lors de l'injection, sous l'effet de la pression d'injection, l'échantillon ECH est filtré par le filtre 14. Les espèces biologiques 4 d'intérêt sont maintenues dans l'espace inférieur 100 de la première chambre 10 et le reste est évacué vers l'extérieur par le premier canal 11.
- E2 : L'étape de lavage (optionnelle) est ensuite mise en œuvre. Un liquide de lavage L est injecté par le deuxième canal 12, à travers le cône 120, pour purifier les espèces biologiques 4 capturées dans l'espace inférieur 100 de la première chambre 10. Ce liquide de lavage L est évacué vers l'extérieur à travers le premier canal 11.
- E3 : Un milieu de culture MC est injecté par le premier canal 11 pour pénétrer dans la première chambre 10.
- E4 : La culture est réalisée dans la première chambre 10. L'étape de culture peut durer 4 heures, en chauffant la première chambre 10 à une température de 37°C.
- E6 : Un liquide de lavage L est injecté par le deuxième canal pour laver les espèces biologiques présentes dans l'espace inférieur de la première chambre.
- E7 Une lyse des espèces biologiques 4 est réalisée dans la première chambre. Cette lyse consiste à broyer les espèces biologiques 4 contre la surface d'appui rugueuse 15 présente au fond de la première chambre 10.
- E8 : Le canal de transfert 22 entre les deux chambres est ouvert. Un réactif d'amplification RA est injecté par le premier canal 11 pour réaliser une élution du matériel biologique 40 obtenu après lyse et transférer le liquide d'intérêt vers la deuxième chambre 20, via le canal de transfert 22.
- Une pression P peut être appliquée à travers le premier canal 11 pour réaliser le transfert du liquide d'intérêt vers la deuxième chambre 20.
- Une réaction d'amplification est mise en œuvre dans la deuxième chambre pendant 45 minutes, en chauffant la deuxième chambre à une température de 60°C. Un dispositif de lecture optique LO est activé pour réaliser une détection de pathogènes dans la deuxième chambre 20. La lecture optique est permise grâce à la transparence des deux parois 200, 201 parallèles délimitant la deuxième chambre.

En référence à la figure 2C, le dispositif peut avantageusement comporter une troisième unité U3, lui permettant de mettre en œuvre le procédé selon la troisième configuration C3 décrite ci-dessus. La troisième unité U3 comporte une troisième chambre 30 de volume non nul, intégrée audit support S. La troisième unité U3 comporte un quatrième canal 31 ménagé dans le support S pour injecter des fluides dans la troisième chambre 30 ou pour évacuer des fluides en dehors de cette troisième chambre 30. Le quatrième canal 31 comporte une première extrémité présentant une ouverture ménagée par exemple à travers une paroi supérieure 12 du support et une deuxième extrémité qui débouche dans ladite troisième chambre 30. La première extrémité du premier canal 31 est par exemple agencée verticalement et sa deuxième extrémité débouche par exemple horizontalement dans la troisième chambre 30. La première extrémité de ce quatrième canal 31 est par exemple obturée par une membrane 310 hydrophobe, c'est-à-dire imperméable au liquide mais perméable au gaz tel que l'air. Cette membrane hydrophobe peut être réalisée dans un matériau de type PTFE (Polytétrafluoroéthylène).

Deux parois du support, avantageusement une paroi supérieure 300 et une paroi inférieure 301, délimitant la troisième chambre 30, sont réalisées dans un matériau transparent, permettant ainsi de réaliser une lecture optique à travers cette troisième chambre. Par le terme "transparent", on entend que le matériau employé est au moins partiellement transparent à la lumière visible, de manière à laisser passer au moins 80% de cette lumière. Il faut ainsi comprendre qu'il sera suffisamment transparent pour voir l'intérieur de la chambre. La paroi inférieure peut être réalisée en verre et la paroi supérieure peut être formée d'un adhésif amovible collé pour fermer ladite troisième chambre du côté supérieur.

Selon une particularité de l'invention, dans cette variante de la figure 2C, le dispositif comporte également un deuxième canal de transfert 32 ménagé dans ledit support S. Ce deuxième canal de transfert 32 est destiné à relier la première chambre 10, plus précisément son espace supérieur 101, à la troisième chambre 30.

De manière avantageuse, le deuxième canal de transfert 32 comporte une première extrémité débouchant directement dans le premier canal 11, formant ainsi un nœud de dérivation sur ce premier canal 11 (de manière symétrique au premier canal de transfert). Il comporte une deuxième extrémité débouchant directement dans la troisième chambre 30.

Comme pour le premier canal de transfert, le dispositif comporte en outre des moyens d'aiguillage pouvant être par exemple agencés sur le premier canal 11 pour sélectionner la liaison de la première chambre :
- vers l'extérieur uniquement via le premier canal ou,
- vers la troisième chambre uniquement à travers le deuxième canal de transfert.

Ces moyens d'aiguillage peuvent consister également en un cône 110 amovible creux se présentant sous la forme d'un entonnoir. Lorsqu'il est inséré dans le premier canal 11, il permet la communication entre l'extérieur et la première chambre 10 et sa paroi obture l'entrée du deuxième canal de transfert 32, réalisée au niveau du nœud de dérivation. Lorsqu'il est retiré, la première extrémité du premier canal 12 est obturée, par exemple à l'aide d'un adhésif 111 appliqué sur une surface du support et la liaison entre le deuxième canal de transfert 32 et le premier canal 11 est alors ouverte, permettant à un fluide de circuler entre la première chambre 10 et la troisième chambre 30.

Comme pour les premiers moyens d'aiguillage, il est entendu que d'autres moyens peuvent être employés, l'objectif étant de disposer d'une solution pour accéder à la première chambre, en obturant le deuxième canal de transfert ou pour autoriser la liaison de la première chambre vers la troisième chambre, à travers le deuxième canal de transfert. Les moyens d'aiguillage de type clapet à deux positions, décrits ci-dessus, peuvent être employés de manière identique.

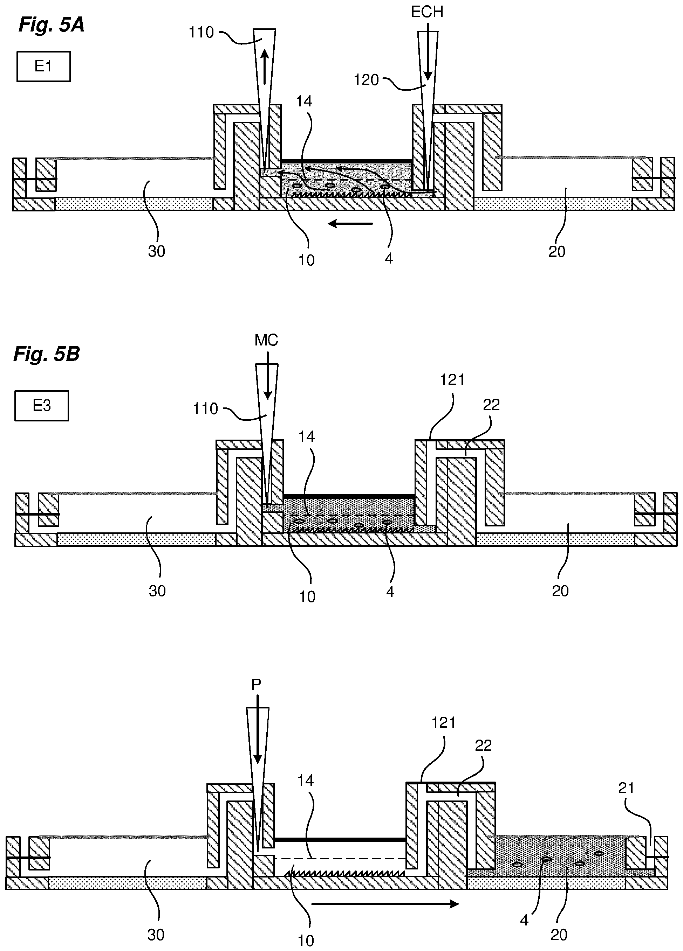

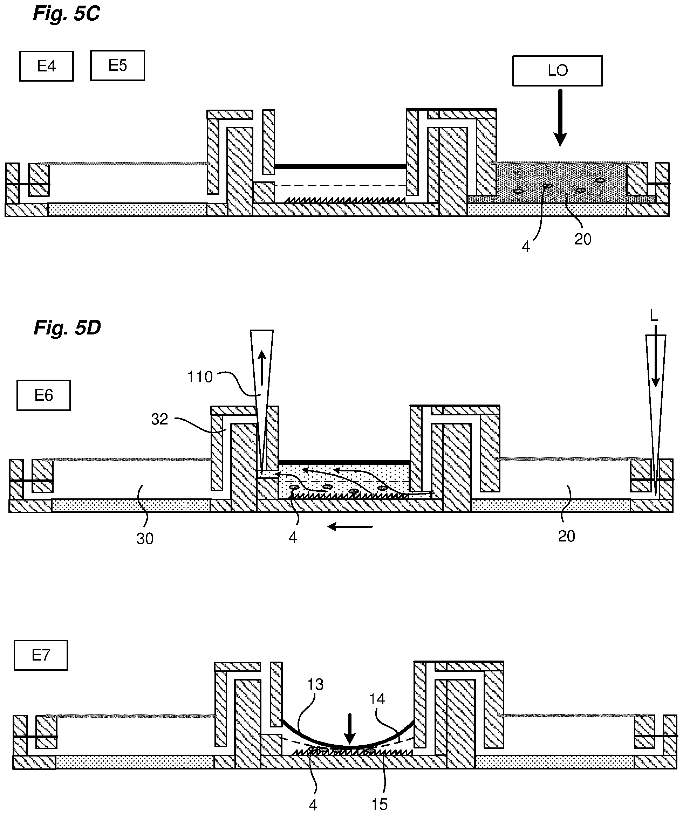

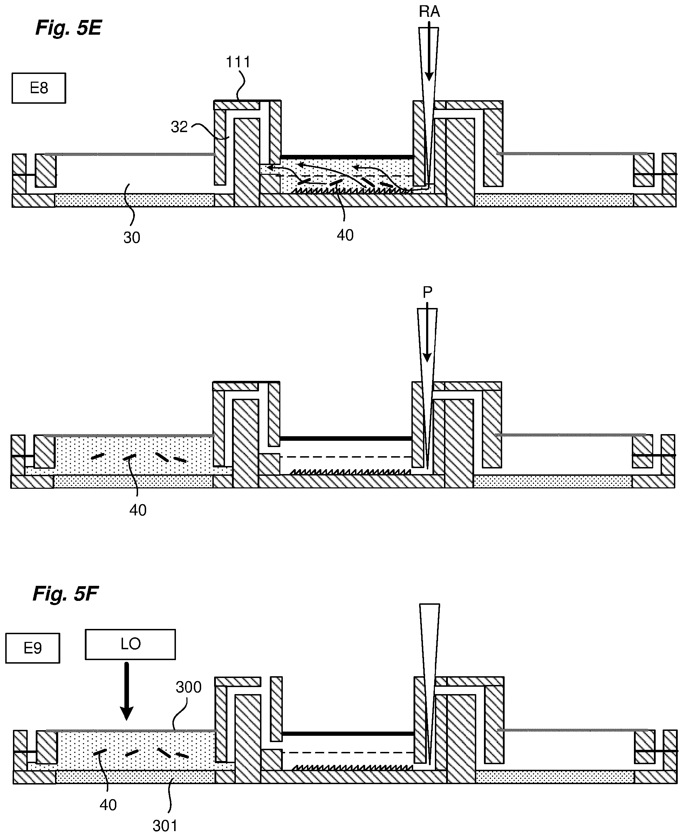

Dans la troisième configuration C3 du procédé mise en œuvre dans le dispositif de la figure 2C et en référence aux figures 5A à 5F, les étapes sont alors les suivantes :
- E1 : L'échantillon liquide ECH est injecté par le deuxième canal 12, par exemple à l'aide d'une pipette à travers le cône 120 vers la première chambre 10. Le cône 120 est agencé dans le deuxième canal 12 à une profondeur suffisante pour obturer l'entrée du premier canal de transfert 22, au niveau du nœud de dérivation. Dans le même temps, le premier canal 11 est ouvert sur l'extérieur pour remplir le rôle d'évent lors du remplissage de la première chambre 10 et pour évacuer le liquide filtré. Lors de l'injection, sous l'effet de la pression d'injection, l'échantillon ECH est filtré par le filtre 14. Les espèces biologiques 4 d'intérêt sont maintenues dans l'espace inférieur 100 de la première chambre 10 et le reste est évacué vers l'extérieur par le premier canal 11.
- L'étape de lavage (optionnelle-non représentée) est ensuite mise en œuvre.

Un liquide de lavage est injecté par le deuxième canal, à travers le cône, pour purifier les espèces biologiques capturées dans l'espace inférieur de la première chambre. Ce liquide de lavage est évacué à travers le premier canal.
- E3 : Un milieu de culture MC est injecté par le premier canal 11 pour pénétrer dans la première chambre 10. L'entrée/sortie du deuxième canal 12 est fermée par l'adhésif 121 et le premier canal de transfert 22 est ouvert, permettant une communication étanche, sans risque de contamination, entre la première chambre 10 et la deuxième chambre 20.
- E3 : Les espèces biologiques 4 dans leur milieu de culture MC sont transférées vers la deuxième chambre 20 via le premier canal de transfert 22. Pour le transfert, une pression d'air P peut être appliquée dans le premier canal. Le troisième canal 21 est obturé grâce à la membrane 210 hydrophobe, permettant à l'air de s'évacuer lors du transfert et d'éviter au liquide de s'échapper.
- E4 - E5 : L'étape de culture peut durer 4 heures, en chauffant la première chambre à une température de 37°C. Un suivi optique de la culture est mis en œuvre à travers la deuxième chambre 20, à l'aide d'un dispositif de lecture optique LO. Cette première étape de lecture permet de s'assurer que la culture est efficace. A titre d'exemple, si dans le cas d'un test de stérilité, aucune bactérie n'est détectée en culture dans la deuxième chambre 20, il est inutile de poursuivre le procédé et de réaliser la purification et l'amplification de l'ADN bactérien. Cette démarche permet alors de minimiser le coût global de l'analyse.
- E6 : Si l'analyse est poursuivie, un liquide de lavage L est injecté par le troisième canal 21 pour laver les espèces biologiques 4 présentes dans la deuxième chambre 20 et pour transférer l'échantillon vers la première chambre 10, à travers le premier canal de transfert 22. Le cône 110 est en place pour fermer l'accès à la troisième chambre 30 via le deuxième canal de transfert 32.
- E7 : Une lyse des espèces biologiques 4 est réalisée dans la première chambre 10. Cette lyse consiste à broyer les espèces biologiques 4 contre la surface d'appui rugueuse 15 présente au fond de la première chambre 10.
- E8 : Le deuxième canal de transfert 32 est ouvert. Un réactif d'amplification RA est injecté par le deuxième canal 12 pour réaliser une élution du matériel biologique obtenu après lyse et transférer le liquide d'intérêt de la première chambre 10 vers la troisième chambre 30.
- Une pression P peut être appliquée à travers le deuxième canal pour réaliser le transfert du liquide d'intérêt vers la troisième chambre.
- Une réaction d'amplification est mise en œuvre dans la troisième chambre pendant 45 minutes, en chauffant la deuxième chambre à une température de 60°C. Un dispositif de lecture optique est activé pour réaliser une détection de pathogènes dans la troisième chambre. La lecture optique est permise grâce à la transparence des deux parois parallèles délimitant la troisième chambre.

On comprend mieux la polyvalence du dispositif de l'invention, que ce soit dans son architecture à deux unités U1, U2 ou à trois unités U1, U2, U3.

En résumé :
- Dans la première configuration C1 du procédé ou dans la configuration C1', la première unité U1 peut être employée pour les étapes E1, E2 et E3 et la deuxième unité U2 pour les étapes E4 et E5.
- Dans la deuxième configuration C2 du procédé ou dans la configuration C2', la première unité U1 peut être employée pour les étapes E1, E2, E3, E4, E6, E7 et E8 et la deuxième unité U2 pour l'étape E9.
- Dans la troisième configuration C3 du procédé, la première unité U1 peut être employée pour les étapes E1, E3, E6, E7 et E8, la deuxième unité U2 pour les étapes E4 et E5 et la troisième unité pour l'étape E9.
- Dans la configuration C4 du procédé, la première unité U1 peut être employée pour les étapes E1, E6, E7, E8 et la deuxième unité pour l'étape E9.

Les deux canaux de transfert permettent de passer aisément d'une unité à une autre, tout en évitant une contamination.

Le dispositif peut avantageusement intégrer des moyens de chauffage de l'espace interne de chaque chambre, composés par exemple d'au moins une résistance 19 chauffante ou d'un élément Peltier, comme représenté sur les figures annexées. La résistance est par exemple fixée sous la paroi inférieure du boîtier. Une source d'alimentation 20 sera par exemple prévue pour alimenter la résistance 19. La source d'alimentation comportera par exemple une ou plusieurs piles électriques, fournissant suffisamment d'énergie pour chauffer la chambre à une température comprise dans la fourchette définie ci-dessus, c'est-à-dire de 20°C à 100°C. Bien entendu, d'autres moyens de chauffage pourraient être employés, comprenant par exemple une encre conductrice déposée par imprimerie ou sérigraphie sous la paroi inférieure du boîtier. Ces moyens de chauffage sont employés pour chauffer la chambre à une température donnée lors d'une étape de culture des espèces biologiques ou lors d'une réaction d'amplification.

De manière non limitative, dans sa variante de la figure 2B, le dispositif peut être réalisé selon l'architecture représentée sur la figure 6.

Sur cette figure 6, le support S comporte les particularités suivantes :
- Le support comporte une lame 50, par exemple en verre ou en en matériau plastique, de type PMMA ou COC ;
- La lame 50 est recouverte dans une première zone d'une surface abrasive de manière à former sur une partie de sa face supérieure la surface d'appui rugueuse 15 dédiée à la lyse ;
- Dans au moins une deuxième zone la lame 50 est transparente pour former la paroi inférieure 201 transparente de la deuxième chambre 20, destinée à la lecture optique ;
- Une première couche 51 portant une première empreinte micro-fluidique est déposée sur la face supérieure de la lame 50, cette première empreinte comportant une première cavité définissant l'espace inférieur 100 de la première chambre 10, une deuxième cavité définissant une partie inférieure de la deuxième chambre 20 et la partie inférieure du troisième canal 21 ; la première cavité a ses bords agencés autour de la première zone abrasive et la deuxième cavité a ses bords agencés autour de la deuxième zone de lecture ;
- Le filtre 14 est apposé sur la première couche pour recouvrir l'espace inférieur 100 de la première chambre et la membrane hydrophobe 210 est apposée sur la partie inférieure du troisième canal 21 ;
- Une deuxième couche 52 portant une deuxième empreinte micro-fluidique est déposée sur la face supérieure de la première couche 51, en recouvrant également le filtre 14 et la membrane hydrophobe 210. Cette deuxième empreinte micro-fluidique comporte une cavité formant l'espace supérieur 101 de la première chambre 10, une deuxième cavité formant une partie médiane de la deuxième chambre 20 et la partie supérieure du troisième canal 21 ;
- Une troisième couche 53 portant une troisième empreinte micro-fluidique est déposée sur la face supérieure de la deuxième couche 52 ; Cette troisième empreinte micro-fluidique comporte la partie supérieure de la première chambre 10 et la partie supérieure de la deuxième chambre 20 ;
- Une lame de verre ou en matériau plastique est dimensionnée pour recouvrir la face supérieure de la troisième couche 53 au niveau de la deuxième chambre, formant la paroi supérieure 200 transparente du support ;
- Un capot 54, par exemple en PMMA, est positionné au-dessus de la première chambre 10 ; Sur sa face inférieure, le capot comporte la membrane 13 destinée à refermer la première chambre par le dessus ;
- Le capot comporte une ouverture axiale supérieure, permettant d'accéder à la membrane 13, pour effectuer la lyse ;
- Le capot 54 comporte deux entrées/sorties fluidiques sur sa face supérieure. La première entrée/sortie est reliée à un premier canal axial traversant formé à travers la membrane 13 et les trois couches et débouchant dans la première chambre 10 pour former le premier canal 11 du support ; La deuxième entrée/sortie est reliée à un deuxième canal axial traversant formé à travers la membrane 13 et la deuxième et troisième couches et débouchant au-dessus de la membrane hydrophobe 210, pour former le troisième canal 21 du support ;
- Le support comporte enfin deux autres canaux traversants formant le premier canal de transfert 22 reliant les deux chambres 10, 20 ;
- Deux autres couches 55, 56 permettent de réaliser l'entrée du deuxième canal 12 et la jonction du canal de transfert 22 sur ce deuxième canal 12 ;

Dans la variante à trois chambres de la figure 2C, l'architecture du support serait similaire, les principes de réalisation de la troisième chambre 30 et du deuxième canal de transfert 32 étant calqués sur ceux de la deuxième chambre 20 et du premier canal de transfert 22.

De manière avantageuse, la deuxième chambre 20 dans la variante à seulement deux unités (figures 2A et 2B) et la deuxième chambre 20 et/ou la troisième chambre 30 dans la variante à trois unités (figure 2C), présente un volume interne façonné de manière adaptée, soit pour assurer un contrôle fiable de la réaction d'amplification, soit pour identifier plusieurs cibles simultanément. Dans ce dernier cas, la chambre peut être façonnée pour permettre d'identifier plusieurs cibles simultanément. Ces tests, dits multiplex, sont par exemple utilisés pour détecter des groupes d'organismes pathogènes correspondant à des symptômes cliniques proches ou à détecter une bactérie mais aussi ses potentiels gènes de résistance aux antibiotiques.

Dans la suite de la description, nous allons décrire l'architecture d'une chambre 25 dite d'amplification de manière générale. Cette architecture peut s'appliquer à la deuxième chambre 20 et/ou à la troisième chambre 30 du dispositif de l'invention déjà décrit ci-dessus.

Cette chambre d'amplification a été dessinée afin de répondre à plusieurs objectifs :
- Optimiser la fluidique (absence de bulles) ;
- Pousser l'air hors du composant mais retenir le liquide ;
- Ne pas contaminer l'environnement ;
- Permettre le multiplexage ;
- Accueillir un contrôle interne de réaction ;
- Limiter les volumes morts ;
- Ne pas diviser l'échantillon ;

Le principe est de créer au moins une alcôve Ax (x allant de 1 à N, N correspondant au nombre d'alcôves et étant supérieur ou égal à 1) dans la chambre d'amplification, pour y loger un composé de contrôle interne de la réaction (par exemple une séquence d'ADN choisie ou des amorces d'amplification visant un ADN prédéfini) adapté à la technologie d'amplification mise en œuvre (PCR, LAMP RPA...). Selon ce principe, soit l'ADN est séché dans l'alcôve, les amorces sont alors amenées par le liquide introduit par la chambre, soit les amorces sont séchées dans l'alcôve et l'ADN est amené par le liquide introduit dans la chambre.

Dans le cas d'un contrôle interne de réaction, le composé de contrôle interne peut être déposée dans l'alcôve Ax à une concentration connue puis séchée directement dans la chambre. Elle reste ainsi à demeure dans le dispositif et est prête à l'emploi.

L'intérêt de déposer le composé de contrôle non pas directement dans la zone de lecture optique mais en dehors de celle-ci permet, en plus d'optimiser l'espace d'échange gazeux, de différencier l'amplification de contrôle de l'amplification cible. L'amplification de contrôle sera en effet à la fois décalée temporellement et spatialement.

L'architecture du dispositif en plusieurs couches permet de réaliser la chambre d'amplification avec des strates de designs différents. La strate inférieure peut en effet comporter une ou plusieurs alcôves et les strates supérieures de la chambre définissent la section transversale totale de lecture optique de la chambre. Ce principe en plusieurs strates est illustré par la figure 7. Sur cette figure 7, on peut voir une strate inférieure S1 portant l'alcôve A1 et une strate supérieure S2 définissant la section transversale de lecture optique (représentée ici de manière non limitative avec une forme rectangulaire). L'alcôve A1 est ici en forme de demi-lune. On comprend de ce principe que le liquide pénètre dans toute la chambre et qu'il peut se répandre dans tout le volume défini par les plusieurs strates, permettant de ne pas perturber la lecture optique et d'obtenir le décalage spatial et temporel décrit ci-dessus.

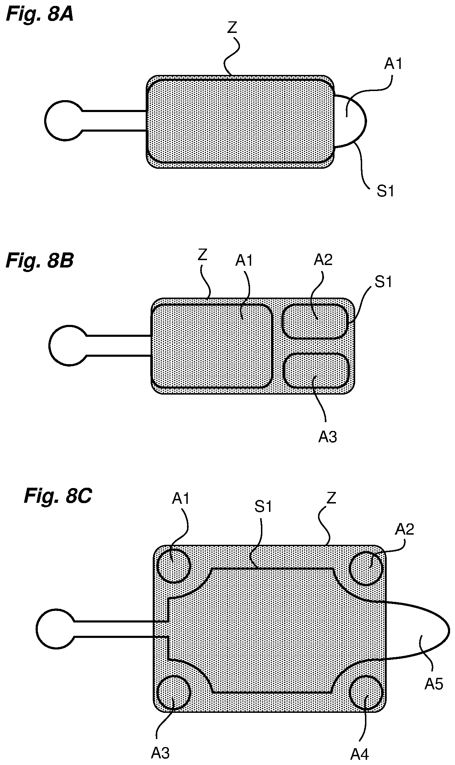

Les figures 8A à 8C montrent différentes architectures de la strate de la chambre d'amplification. Sur ces figures, la zone Z de lecture optique est représentée en grisé. Sur la figure 8A, la strate inférieure S1 comporte une proéminence formant l'alcôve A1 allant au-delà de la section de la zone Z de lecture optique de la chambre dans le plan transversal, à l'opposé du point d'entrée de liquide dans la chambre (correspondant au design de la figure 7). Cette zone déportée permet de décaler la réaction d'amplification temporellement et spatialement. Sur la figure 8B, la strate inférieure S1 comporte trois alcôves A1, A2, A3 distinctes permettant de loger trois amorces distinctes. Ces trois zones sont situées dans la zone Z de lecture optique et permettent de mettre en œuvre le principe de multiplexage. La figure 8C montre une state inférieure, définissant cinq zones distinctes, quatre alcôves A1-A4 réalisées chacune sous la forme d'une cavité et une zone principale comprenant une proéminence présentant une cinquième alcôve A5. La strate de la chambre qui est située juste au-dessus définit la zone de lecture optique. La zone Z de lecture optique présente un design recouvrant les quatre alcôves A1-A4 pour que son volume soit en communication fluidique avec celles-ci. La cinquième alcôve peut être en dehors de la zone Z de lecture optique, comme dans le design de la figure 8A.

Comme décrit ci-dessus chaque amorce peut être placé dans une alcôve distincte de la chambre, sous une forme sèche. Pour faciliter le séchage il est conseillé de reprendre les amorces dans un tampon acide (environ pH 6) et faciliter leur liaison avec le verre de la lame. Il est aussi possible d'utiliser des sucres (exemple tréhalose) pour limiter la diffusion de la goutte et potentiellement augmenter la stabilité de la séquence d'ADN séchée.

On comprend des éléments ci-dessus que le dispositif de l'invention présente de nombreux avantages, parmi lesquels :
- Une polyvalence qui lui permet de mettre en œuvre de manière fiable certaines étapes du procédé de préparation et d'analyse d'un échantillon biologique ou toutes les étapes de ce procédé ;
- Un dispositif facilement transportable et manipulable :
- Un dispositif monobloc, permettant de réaliser toutes les étapes dans un unique support micro-fluidique, sans transfert d'échantillon et donc sans risque de contamination ;
- Un dispositif qui présente une solution de contrôle de réaction, grâce au design particulier de sa chambre d'amplification.

## Revendications

1. Dispositif micro-fluidique de préparation et d'analyse d'un échantillon biologique contenant des espèces biologiques (4), ledit dispositif comprenant :
- Un unique support (S) rigide,
- Une première unité (U1) comprenant une première chambre (10) de volume non nul délimitée par des parois du support, un filtre (14) séparant ladite première chambre (10) en un premier espace (101) et un deuxième espace (100), un premier canal (11) réalisé dans ledit support débouchant à une extrémité sur une surface dudit support et à une autre extrémité dans ledit premier espace (101) et un deuxième canal (12) réalisé dans ledit support et débouchant à une extrémité sur une surface dudit support et à une autre extrémité dans ledit deuxième espace (100),
**caractérisé en ce que** le dispositif comporte :
- Une deuxième unité (U2) comprenant une deuxième chambre (20) réalisée dans ledit support (S) rigide et délimitée au moins partiellement par une paroi (200, 201) transparente dudit support (S), un troisième canal (21) réalisé dans ledit support (S) et débouchant à une extrémité sur une surface dudit support et à une autre extrémité dans ladite deuxième chambre (20),
- Un premier canal de transfert (22) fluidique entre la première chambre (10) et la deuxième chambre (20), réalisé dans ledit support (S) et débouchant d'une part dans ladite deuxième chambre (20) et d'autre part en un premier nœud de dérivation dans ledit deuxième canal (12),
- Des premiers moyens d'aiguillage de flux agencés pour sélectionner la liaison de la première chambre :
- vers l'extérieur uniquement via le deuxième canal uniquement, en obturant l'embouchure du premier canal de transfert (22) au niveau du premier nœud de dérivation ou,
- vers la deuxième chambre uniquement à travers le premier canal de transfert en ouvrant la liaison entre le deuxième canal (12) et le deuxième canal de transfert (22) au niveau du premier nœud de dérivation.

2. Dispositif selon la revendication 1, **caractérisé en ce que** la première unité comporte une surface d'appui rugueuse (15) réalisée au fond de sa première chambre (10).

3. Dispositif selon la revendication 1 ou 2, **caractérisé en ce que** la première chambre est fermée par une membrane (13) déformable.

4. Dispositif selon l'une des revendications 1 à 3, **caractérisé en ce qu'**il comporte une première membrane (210) hydrophobe obturant le troisième canal (21).

5. Dispositif selon l'une des revendications 1 à 4, **caractérisé en ce qu'**il comporte :
- Une troisième unité (U3) comprenant une troisième chambre (30) réalisée dans ledit support (S) rigide et délimitée au moins partiellement par une paroi (300, 301) transparente dudit support (S), un quatrième canal (31) réalisé dans ledit support (S) et débouchant à une extrémité sur une surface dudit support et à une autre extrémité dans ladite troisième chambre (30).

6. Dispositif selon la revendication 5, **caractérisé en ce qu'**il comporte :
- Un deuxième canal de transfert (32) fluidique entre la première chambre (10) et la troisième chambre (30), réalisé dans ledit support (S) et débouchant d'une part dans ladite troisième chambre (30) et d'autre part en un deuxième nœud de dérivation dans ledit premier canal (11).

7. Dispositif selon la revendication 6, **caractérisé en ce qu'**il comporte des deuxièmes moyens d'aiguillage de flux agencés pour sélectionner la liaison de la première chambre :
- vers l'extérieur uniquement via le premier canal uniquement ou,
- vers la troisième chambre uniquement à travers le deuxième canal de transfert.

8. Dispositif selon l'une des revendications 5 à 7, **caractérisé en ce qu'**il comporte une deuxième membrane (310) hydrophobe obturant le quatrième canal (31).

9. Dispositif selon l'une des revendications 1 à 8, **caractérisé en ce que** la deuxième chambre comporte au moins une alcôve destinée à recevoir un composé de contrôle interne de réaction.

10. Dispositif selon la revendication 9, **caractérisé en ce que** la deuxième chambre est réalisée en plusieurs strates superposées et **en ce que** ladite alcôve est réalisée dans l'une desdites strates uniquement.

11. Procédé de préparation et d'analyse d'un échantillon biologique contenant des espèces biologiques, **caractérisé en ce qu'**il est mis en œuvre à l'aide d'un dispositif micro-fluidique tel que défini dans l'une des revendications 5 à 8, ou tel que défini dans l'une des revendications 9 à 10 lorsque la revendication 9 est dépendante de l'une des revendications 5 à 8, et **en ce que** :
- La première unité (U1) est configurée pour mettre en œuvre une ou plusieurs des étapes suivantes dudit procédé :
> Concentration des espèces biologiques présentes dans un échantillon biologique,
> Lavage pour purification des espèces biologiques,
> Réception d'un milieu de culture,
> Culture des espèces biologiques,
> Lyse des espèces biologiques en vue de libérer un matériel biologique,
> Séparation du matériel biologique,
- La deuxième unité (U2) est configurée pour mettre en œuvre une ou plusieurs des étapes suivantes dudit procédé :
> Culture des espèces biologiques,
> Suivi optique de croissance lors de ladite étape de culture,
> Détection de présence de pathogènes dans le matériel biologique séparé, par amplification biomoléculaire,
- La troisième unité (U3) est configurée pour mettre en œuvre l'étape suivante dudit procédé :
> Détection de présence de pathogènes dans le matériel biologique séparé, par amplification biomoléculaire.

## Patentansprüche

1. Mikrofluidikvorrichtung zur Vorbereitung und Analyse einer biologischen Probe, die biologische Arten (4) enthält, wobei die Vorrichtung Folgendes umfasst:
- einen starren Einzelträger (S),
- eine erste Einheit (U1) umfassend eine erste Kammer (10) eines Volumens ungleich null, die von Wänden des Trägers begrenzt ist, einen Filter (14), der die erste Kammer (10) in einen ersten Raum (101) und einen zweiten Raum (100) trennt, einen ersten Kanal (11), der im Träger ausgebildet ist, der an einem Ende auf einer Fläche des Träger und an einem anderen Ende im ersten Raum (101) mündet, und einen zweiten Kanal (12), der im Träger ausgebildet ist und an einem Ende auf einer Fläche des Trägers und an einem anderen Ende im zweiten Raum (100) mündet,
**dadurch gekennzeichnet, dass** die Vorrichtung Folgendes aufweist:
- eine zweite Einheit (U2) umfassend eine zweite Kammer (20), die im starren Träger (S) ausgebildet ist und wenigstens teilweise von einer transparenten Wand (200, 201) des Trägers (S) begrenzt ist, einen dritten Kanal (21), der im Träger (S) ausgebildet ist und an einem Ende auf einer Fläche des Trägers und an einem anderen Ende in der zweiten Kammer (20) mündet,
- einen ersten Fluidübertragungskanal (22) zwischen der ersten Kammer (10) und der zweiten Kammer (20), der im Träger (S) ausgebildet ist und einerseits in der zweiten Kammer (20) und andererseits in einem ersten Abzweigknoten im zweiten Kanal (12) mündet,
- erste Flusslenkungsmittel, die angeordnet sind, die Verbindung der ersten Kammer wie folgt auszuwählen:
- nur nach außen nur über den zweiten Kanal durch Verschluss der Mündung des ersten Übertragungskanals (22) am ersten Abzweigknoten, oder
- zur zweiten Kammer nur über den ersten Übertragungskanal durch Öffnen der Verbindung zwischen dem zweiten Kanal (12) und dem zweiten Übertragungskanal (22) am ersten Abzweigknoten.

2. Vorrichtung nach Anspruch 1, **dadurch gekennzeichnet, dass** die erste Einheit eine raue Auflagefläche (15) aufweist, die am Boden ihrer ersten Kammer (10) ausgebildet ist.

3. Vorrichtung nach Anspruch 1 oder 2, **dadurch gekennzeichnet, dass** die erste Kammer von einer verformbaren Membran (13) verschlossen ist.

4. Vorrichtung nach einem der Ansprüche 1 bis 3, **dadurch gekennzeichnet, dass** sie eine erste hydrophobe Membran (210) aufweist, die den dritten Kanal (21) verschließt.

5. Vorrichtung nach einem der Ansprüche 1 bis 4, **dadurch gekennzeichnet, dass** sie Folgendes aufweist:
- eine dritte Einheit (U3) umfassend eine dritte Kammer (30), die im starren Träger (S) ausgebildet ist und wenigstens teilweise von einer transparenten Wand (300, 301) des Trägers (S) begrenzt ist, einen vierten Kanal (31), der im Träger (S) ausgebildet ist und an einem Ende auf einer Fläche des Trägers und an einem anderen Ende in der dritten Kammer (30) mündet.

6. Vorrichtung nach Anspruch 5, **dadurch gekennzeichnet, dass** sie Folgendes aufweist:
- einen zweiten Fluidübertragungskanal (32) zwischen der ersten Kammer (10) und der dritten Kammer (30), der im Träger (S) ausgebildet ist und einerseits in der dritten Kammer (30) und andererseits in einem zweiten Abzweigknoten im ersten Kanal (11) mündet.

7. Vorrichtung nach Anspruch 6, **dadurch gekennzeichnet, dass** sie zweite Flusslenkungsmittel aufweist, die angeordnet sind, die Verbindung der ersten Kammer wie folgt auszuwählen:
- nur nach außen nur über den ersten Kanal, oder
- zur dritten Kammer nur über den zweiten Übertragungskanal.

8. Vorrichtung nach einem der Ansprüche 5 bis 7, **dadurch gekennzeichnet, dass** sie eine zweite hydrophobe Membran (310) aufweist, die den vierten Kanal (31) verschließt.

9. Vorrichtung nach einem der Ansprüche 1 bis 8, **dadurch gekennzeichnet, dass** die zweite Kammer mindestens einen Alkoven aufweist, der dazu bestimmt ist, eine Zusammensetzung zur internen Reaktionssteuerung aufzunehmen.

10. Vorrichtung nach Anspruch 9, **dadurch gekennzeichnet, dass** die zweite Kammer in mehreren übereinander gelagerten Schichten ausgebildet ist, und dadurch, dass der Alkoven nur in einer der Schichten ausgebildet ist.

11. Verfahren zur Vorbereitung und Analyse einer biologischen Probe, die biologische Arten enthält, **dadurch gekennzeichnet, dass** es mithilfe einer mikrofluidischen Vorrichtung nach einem der Ansprüche 5 bis 8 oder nach einem der Ansprüche 9 bis 10, wenn Anspruch 9 von einem der Ansprüche 5 bis 8 abhängig ist, durchgeführt wird, und dadurch, dass:
- die erste Einheit (U1) dazu ausgebildet ist, einen oder mehrere der folgenden Schritte des Verfahrens durchzuführen:
> Konzentrieren der biologischen Arten, die in einer biologischen Probe vorhanden sind,
> Waschen zur Reinigung der biologischen Arten,
> Empfangen eines Kulturmediums,
> Kultivieren der biologischen Arten,
> Lyse der biologischen Arten zur Freisetzung eines biologischen Materials,
> Trennen des biologischen Materials,
- die zweite Einheit (U2) dazu ausgebildet ist, einen oder mehrere der folgenden Schritte des Verfahrens durchzuführen:
> Kultivieren der biologischen Arten,
> optisches Verfolgen des Wachstums beim Schritt des Kultivierens,
> Erkennen des Vorhandenseins von Pathogenen im getrennten biologischen Material durch biomolekulare Verstärkung,
- die dritte Einheit (U3) dazu ausgebildet ist, den folgenden Schritt des Verfahrens durchzuführen:
> Erkennen des Vorhandenseins von Pathogenen im getrennten biologischen Material durch biomolekulare Verstärkung.

## Claims

1. Microfluidic device for preparing and analyzing a biological sample containing biological species (4), said device comprising:
- a single rigid support (S),
- a first unit (U1) comprising a first chamber (10) of nonzero volume delimited by walls of the support, a filter (14) separating said first chamber (10) into a first space (101) and a second space (100), a first channel (11) made in said support opening at one end onto a surface of said support and at another end into said first space (101) and a second channel (12) made in said support and opening at one end onto a surface of said support and at another end into said second space (100),
**characterized in that** the device has:
- a second unit (U2) comprising a second chamber (20) made in said rigid support (S) and delimited at least partially by a transparent wall (200, 201) of said support (S), a third channel (21) made in said support (S) and opening at one end onto a surface of said support and at another end into said second chamber (20),
- a first fluidic transfer channel (22) between the first chamber (10) and the second chamber (20), made in said support (S) and opening on the one hand into said second chamber (20) and on the other hand at a first bypass node in said second channel (12),
- first flow switching means arranged for selecting connection of the first chamber:
- to the exterior only, via the second channel only, by sealing the mouth of the first transfer channel (22) at the level of the first bypass node, or
- to the second chamber only, through the first transfer channel, by opening the connection between the second channel (12) and the second transfer channel (22) at the level of the first bypass node.

2. Device according to Claim 1, **characterized in that** the first unit comprises a rough contact surface (15) made at the bottom of its first chamber (10).

3. Device according to Claim 1 or 2, **characterized in that** the first chamber is closed by a deformable membrane (13).

4. Device according to one of Claims 1 to 3, **characterized in that** it comprises a first hydrophobic membrane (210) sealing the third channel (21).

5. Device according to one of Claims 1 to 4, **characterized in that** it comprises:
- a third unit (U3) comprising a third chamber (30) made in said rigid support (S) and delimited at least partially by a transparent wall (300, 301) of said support (S), a fourth channel (31) made in said support (S) and opening at one end onto a surface of said support and at another end into said third chamber (30).

6. Device according to Claim 5, **characterized in that** it comprises:
- a second fluidic transfer channel (32) between the first chamber (10) and the third chamber (30), made in said support (S) and opening on the one hand into said third chamber (30) and on the other hand at a second bypass node into said first channel (11).

7. Device according to Claim 6, **characterized in that** it comprises second flow switching means arranged for selecting connection of the first chamber:
- to the exterior only via the first channel only or,
- to the third chamber only through the second transfer channel.

8. Device according to one of Claims 5 to 7, **characterized in that** it comprises a second hydrophobic membrane (310) sealing the fourth channel (31).

9. Device according to one of Claims 1 to 8, **characterized in that** the second chamber comprises at least one recess intended to receive a compound for internal reaction control.

10. Device according to Claim 9, **characterized in that** the second chamber is made up of several superposed strata and **in that** said recess is made in one of said strata only.

11. Method for preparing and analyzing a biological sample containing biological species, **characterized in that** it is carried out by means of a microfluidic device as defined in one of Claims 5 to 8, or as defined in either of Claims 9 and 10 when Claim 9 is dependent on one of Claims 5 to 8, and **in that**:
- the first unit (U1) is configured for carrying out one or more of the following steps of said method:
> concentrating the biological species present in a biological sample,
> washing to purify the biological species,
> receiving a culture medium,
> culturing the biological species,
> lysis of the biological species in order to release a biological material,
> separating the biological material,
- The second unit (U2) is configured for carrying out one or more of the following steps of said method:
> culturing the biological species,
> visual monitoring of growth during said culture step,
> detecting the presence of pathogens in the separated biological material, by biomolecular amplification,
- The third unit (U3) is configured for carrying out the following step of said method:
> detecting the presence of pathogens in the separated biological material, by biomolecular amplification.
